# DEMANDE DE BREVET EUROPEEN

(11) **EP 0 671 173 A1**
(43) Date de publication de la demande: **13.09.1995**
(21) Numéro de dépôt: 95400383.6
(22) Date de dépôt: 23.02.1995
(51) Int. Cl.: A61K 31/55

(54) **Utilisation d'un dérivé tricyclique pour l'obtention de médicaments destinés au traitement des troubles mnémo-cognitifs**

(30) Priorité: 25.02.1994 FR 9402160
(71) Demandeur: ADIR ET COMPAGNIE, F-92415 Courbevoie Cédex (FR)
(72) Inventeur: Kamoun, Annie, F-92200 Neuilly Sur Seine (FR); Delalleau, Bruno, F-92400 Courbevoie (FR); Deslandes, Antoine, F-75007 Paris (FR)

(57) **Abrégé**

L'invention concerne l'utilisation d'un dérivé tricyclique pour l'obtention de médicaments destinés au traitement des troubles mnémo-cognitifs.

Médicaments.

## Description

La présente invention concerne l'utilisation d'un dérivé tricyclique pour l'obtention de médicaments destinés au traitement des troubles mnémo-cognitifs.

Plus spécifiquement, l'invention concerne l'utilisation, pour l'obtention de médicaments destinés aux troubles mnémo-cognitifs, du composé de formule (I) : 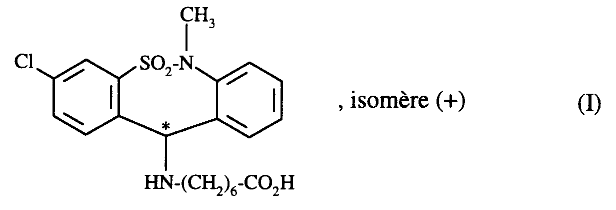
ainsi que ses sels d'addition à un acide ou à une base pharmaceutiquement acceptable.

Comme base pouvant salifier les composés de formule (I), on pourra utiliser l'hydroxyde de sodium, potassium, calcium ou d'aluminium, des carbonates de métaux alcalins ou alcalino-terreux ou des bases organiques comme la triéthylamine, la benzylamine, la diéthanolamine, la tert-butylamine, la dicyclohexylamine, l'arginine, etc...

Parmi les acides que l'on peut ajouter aux composés de formule (I) pour obtenir un sel d'addition, on peut citer à titre non limitatif les acides chlorhydrique, sulfurique, phosphorique, tartrique, malique, maléique, fumarique, oxalique, méthane-sulfonique, éthanesulfonique, camphorique, citrique, etc...

Le composé de formule (I) préférentiellement utilisable selon l'invention est l'isomère (+) de la tianeptine sous forme de sel de sodium, ou (+)7-[(3-chloro-6-méthyl-5,5-dioxo-6,11-dihydrodibenzo[c,f]-[1,2]-thiazepin-11-yl)amino]heptanoate de sodium.

La tianeptine est décrite dans le brevet français FR 2104728 (exemple 3). Le brevet FR 2104728 revendique l'utilisation de la tianeptine et de ses dérivés dans le traitement des troubles psychonévrotiques, de la douleur et de la toux. Le brevet français FR 2635461 décrit l'utilisation de la tianeptine et de ses dérivés dans le traitement du stress.

Des travaux récents ont montré que la tianeptine avait des effets significatifs sur la mémoire. Ce sont ceux décrits par :
- S.E. FILE et coll. dans : *Drug Development Research*, 1991, 23, 47-56 ;
- R. Jaffard et coll. dans : *Journal de Psychiatrie Biologique et Thérapeutique*, 1989, n° spécial mars, 37-39 ;
- R. Jaffard et coll. dans : *Abstracts of the XVI*^{*th*}*CINP Congress*, Munich (Germany), 15-19 August 1988; *Psychopharnacology*, 1988, 96 (suppl), 31.02.32, p275
- R. Jaffard et coll. dans : *Behavioural Pharmacology*, 1991, 2, 37-46
- C. Lebrun et coll. dans : *European Psychiatry*, 1993, 8 (suppl.2), 81s-88s
- R. Jaffard et coll. dans : *Presse Médicale*, 1991, 20, 37, 1812-1816.

La demanderesse a maintenant découvert que, de façon surprenante, l'isomère (+) de la tianeptine, présentait des propriétés pharmacologiques nettement supérieures à celles observées pour la tianeptine. Ces propriétés lui permettent d'être utilisé dans le traitement des troubles mnésiques et cognitifs et des troubles neuro-comportementaux associés au vieillissement cérébral et aux maladies dégénératives du système nerveux, aiguës ou chroniques comme la maladie d'Alzheimer, de Pick, le syndrôme de Korsakoff, l'accident vasculaire cérébral, le traumatisme spinal, la sclérose amyotrophique latérale ou la sclérose en plaques.

Les résultats obtenus chez la souris pourraient être obtenues chez l'homme. En effet, si l'on postule que la vitesse de l'oubli est déterminée par des stratégies d'encodage en rapport avec l'hippocampe, il semble qu'une épreuve de mémoire qui mettrait essentiellement en oeuvre la représentation d'expériences successives semblables dans un contexte spécifique, pourrait convenir à l'évaluation des effets du vieillissement sur l'oubli à long terme chez l'homme (C.Lebrun et coll. European Psychiatry, 1993, 8 (suppl.2), 81s-88s). Il semblerait d'autre part, que certains mécanismes cérébraux de base intervenant dans l'oubli à long terme normal et pathologique soient communs à l'homme, au singe et aux rongeurs (Squire, Psychological Review, 1992, 99, 195-231).

D'autre part, I'énantiomère (+) présente un profil toxicologique et pharmacocinétique satisfaisant. Chez le rat, la biodisponibilité de l'isomère (+) est de 29 % alors que la biodisponibilité du racémique n'est que de 11 %. Son absorption est rapide (tₘₐₓ = 0,5 h) après administration orale. Sa demi-vie est de 1,8 h. Enfin, contrairement à l'énantiomère (-) et au racémique qui diminuent de façon dose-dépendante (5, 10 et 15 mg/kg), le nombre de fèces expulsés et les tremblements induits par le 5-HTP, l'énantiomère (+) n'a pas d'effet significatif. Ainsi, l'énantiomère (+) ne présente donc pas d'effet sur la transmission sérotoninergique contrairement au racémique et à l'énantiomère (-).

Les composés de formule (I) ou leurs sels pharmaceutiquement acceptables seront présentés sous des formes pharmaceutiques convenant pour l'administration par voie orale, parentérale et notamment intraveineuse, per ou transcutanée, nasale, rectale, perlinguale, oculaire ou respiratoire et notamment les préparations injectables, les aérosols, les gouttes oculaires, ou nasales, les comprimés, les comprimés sublinguaux, les glossettes, les gélules, les capsules, les tablettes, les suppositoires, les crèmes, pommades, gels dermiques, etc...

La posologie varie selon l'âge et le poids du patient, la voie d'administration, la nature de l'indication thérapeutique et des traitements associés et s'échelonne de 0,1 mg à 100 mg par prise ou par application.

Les exemples suivants illustrent l'invention et ne la limitent en aucune façon.

### Exemple 1 : Synthèse stéréospécifique du (+)7-[(3-chloro-6-méthyl-5,5-dioxo-6,11- dihydrodibenzo[c,f]-[1,2]-thiazepin-11-yl)amino]heptanoate de sodium (ou isomère (+) de la tianeptine)

L'isomère (+) de la tianeptine a été obtenu par dédoublement de la 11-amino-3-chloro-6-méthyl-5,5-dioxo-6,11-dihydrodibenzo[c,f]-[1,2]-thiazépine, condensation de l'isomère avec le 7-bromoheptanoate d'éthyle puis saponification de l'ester obtenu.
Stade A : 11-Amino-3-chloro-6-méthyl-5,5-dioxo-6,11-dihydro-dibenzo[c,f]-[1,2]-thiazepine, (-) dibenzoyltartrate
A 162 mmoles de 11-amino-3-chloro-6-méthyl-5,5-dioxo-6,11-dihydrodibenzo[c,f]-[1,2]-thiazepine en suspension dans 600 ml d'éthanol à 96 %, sont ajoutées 162 mmoles d'acide (-) dibenzoyl tartrique, monohydrate en solution dans 400 ml d'éthanol à 96 %. Le mélange est agité, 3 heures à température ambiante. Après filtration et séchage du précipité obtenu, celui-ci est recristallisé deux fois dans de l'éthanol à 96 % et conduit au produit attendu.
Pureté isomérique : 99 %
Stade B : (+) 7-[(3-Chloro-6-méthyl-5,5-dioxo-6,11-dihydrodibenzo[c,f]-[1,2]-thiazepin-11-yl)amino]heptanoate d'éthyle
43,5 mmoles du composé obtenu au stade précédent sont mélangées avec du carbonate de sodium 0,5 M et extraits par du dichlorométhane. La phase organique est séchée et évaporée. Au solide blanc ainsi obtenu, sont ajoutés 200 ml d'éthanol anhydre puis 10 g de carbonate de potassium anhydre. L'ensemble est porté à reflux et 65,4 mmoles de 7-bromoheptanoate d'éthyle sont ajoutées goutte à goutte. L'ensemble est maintenu 3 jours à reflux, sous atmosphère d'azote, à l'abri de la lumière. Après filtration du milieu réactionnel et évaporation du filtrat, le résidu est repris par de l'éther éthylique. L'insoluble est filtré et le filtrat évaporé. L'huile ainsi obtenue est reprise par de l'éther isopropylique et conduit au produit attendu après filtration et séchage du précipité formé.
Stade C : (+) 7-[(3-Chloro-6-méthyl-5,5-dioxo-6,11-dihydrodibenzo[c,f]-[1,2]-thiazepin-11-yl)amino]heptanoate de sodium
42 mmoles du composé obtenu au stade précédent sont placées dans un mélange contenant 45 ml d'éthanol à 96 % et 45 ml de soude 1N. L'ensemble est agité une nuit à température ambiante, après évaporation de l'éthanol, lavage par du dichlorométhane, la phase aqueuse est neutralisée par 45 ml d'acide chlorhydrique 1N. L'acide correspondant est alors extrait par du dichlorométhane, séché, évaporé et purifié par chromatographie sur colonne de silice en utilisant comme éluant un mélange dichlorométhane/méthanol (98/2). Il est alors transformé en sel de sodium correspondant par dissolution dans une quantité stoechiométrique de soude 1N. Après addition de 50 ml d'eau, le produit attendu est lyophilisé.
Pouvoir rotatoire : [α]_{D}²⁰ = + 9,2° (c = 5,0 mg/ml, Ethanol 95 %)

| Microanalyse élémentaire : | | | |
|---|---|---|---|
| | C % | H % | N % |
| calculé | 54,96 | 5,27 | 6,10 |
| trouvé | 55,08 | 5,49 | 6,16 |

La pureté énantiomérique est vérifiée par chromatographie liquide.
Colonne : L = 15 cm, diamètre intérieur: 4,6 mm
Phase mobile : KH₂PO₄, 20 mM, pH 6,1 / EtOH: 8/2
Débit : 0,9 ml/min
Température : ambiante
Détection : UV = 220 nm
Solution : 0,5 mg/ml dans un tampon phosphate de potassium pH 6,1
Injection : 2 µl

Dans ces conditions la pureté énantiomérique de composé est supérieure à 99 %.

### Exemple 2 : Etude pharmacologique de l'isomère (+) de la tianeptine

Comparaison des effets de la tianeptine et de l'isomère (+) de celle-ci sur l'alternance spontanée, l'acquisition et la rétention d'une discrimination spatiale simple (labyrinthe en T) chez la souris.

Des résultats antérieurs ont montré que la tianeptine facilitait, à la dose de 10 mg/kg, l'alternance spontanée, l'acquisition et la rétention d'une discrimination simple réalisées dans un labyrinthe en T chez la souris. Les effets facilitateurs de la tianeptine avaient été observés lors de deux expériences indépendantes utilisant un appareil identique mais placé dans un environnement différent (pièce), et réalisées par des expérimentateurs différents (Jaffard et coll., Behav. Pharmacol., 1991, 2, 37-46). Le but de cette expérience est, en utilisant les mêmes épreuves, de comparer les effets de la tianeptine à ceux de l'isomère (+).

Le principe du test s'appuie sur la tendance innée qu'ont les mammifères confrontés à un choix entre deux directions à prendre, à aller explorer le lieu qui leur est le moins familier. Un animal placé plusieurs fois successivement à l'entrée d'un labyrinthe en T se dirigera de préférence vers le bras qu'il n'a pas visité précédemment. Pour que ce comportement apparaisse, il est évidemment nécessaire que l'animal se souvienne du dernier bras visité. Ce souvenir met en jeu la mémoire dite de travail. La souris est soumise à une phase d'apprentissage (acquisition) pendant laquelle elle doit choisir plusieurs fois, de suite entre les deux bras, jusq'uà ce qu'elle ait opté cinq fois de suite pour un bras déterminé par l'expérimentateur. Pour apprécier si l'animal a bien retenu ce qu'il a appris au cours de l'épreuve d'acquisition, il est replacé 24 heures plus tard dans la même situation. Le degré de rétention sera mesuré par le nombre d'épreuves subies avant la réussite de cinq essais consécutifs.

### Techniques et méthodes

*Animaux* :
36 souris mâles de la lignée BALB/c, vierges de toute expérimentation et âgées de 14 à 16 semaines, sont utilisées. Elles sont placées, 15 jours avant le début des expériences, dans des cages individuelles (animalerie climatisée (22°C) et pourvue d'un cycle artificiel lumière-obscurité de 12 heures (7h ON)).

*Appareil* :
C'est un labyrinthe en T construit en plexiglas de couleur grise. Le couloir central et les deux bras d'arrivée mesurent 35 cm de long, 10 cm de large et 20 cm de haut. Le compartiment de départ (10 x 12 cm) est séparé du couloir central par une porte à guillotine. L'entrée de chaque bras est également pourvue d'une porte. L'appareil utilisé est semi-automatique : l'alimentation des mangeoires, l'ouverture et la fermeture des portes sont commandées par un micro-ordinateur selon le protocole pré-établi.

*Protocole* :
Toutes les épreuves se déroulent entre 9h et 16h et durant 4 jours (de J1 à J4) sans interruption. La ration alimentaire des animaux est réduite de façon à atteindre un poids corporel de 83-84 % du poids *ad libitum* pour les épreuves d'habituation (J1 et J2) et de 87-88 % lors des épreuves d'alternance et de discrimination spatiale (J3 et J4).

*Habituation (J1-J2)* :
L'expérience proprement dite est précédée de deux séances d'habituation de 10 minutes (exploration libre, une séance par jour). L'animal est simplement placé dans l'appareil toutes portes ouvertes.

*Alternances spontanées et acquisition de la discrimination (J3)* :
Ces deux épreuves ont lieu le lendemain sans interruption. Elles sont destinées à mesurer l'alternance (6 premiers essais) et la vitesse d'acquisition de la discrimination spatiale (essais suivants). Lors des 6 premiers essais, la nourriture est disposée à l'extrémité de chacun des deux bras. Lors de chaque essai, l'animal est placé dans le compartiment de départ pendant 30 secondes (intervalle entre essais). La porte donnant accès au couloir central est ouverte et la souris peut choisir entre les deux bras où elle reste enfermée pendant 30 secondes. On constate que l'animal normal alterne ses choix successifs (65 % d'alternance environ). Apartir du 6ème essai, la nourriture n'est plus disposée que dans un seul bras, toujours le même ; le bras choisi est celui qui a été le moins visité au cours des 6 premiers essais. L'épreuve se poursuit alors jusqu'à ce que l'animal atteigne un critère de 5 bonnes réponses sur 5 essais consécutifs.

*Rétention de la discrimination spatiale (J4)* :
Elle se déroule dans les mêmes conditions que l'acquisition (même bras renforcé) et se poursuit pendant un minimum de 10 essais ou, au-delà, jusqu'à l'atteinte du critère de 5/5. Le nombre d'essais est limité à 20.

*Traitements et Groupes* :
Les 36 animaux sont divisés en 3 groupes de 12 sujets recevant, selon le groupe, l'un des produits testés lors de la réalisation de l'expérience: la tianeptine, l'isomère (+) ou le placebo. Les traitements sont administrés par voie intra-péritonéale 30 minutes avant les épreuves du jour 3 (alternance + acquisition) et du jour 4 (rétention) à la dose de 10 mg/kg pour chacun des produits. Le groupe témoin reçoit le solvant (0,1 ml/10 g de sérum physiologique).

*Analyse des résultats* :
Toutes les valeurs sont exprimées en moyennes affectées de leur erreur standard. Les résultats portant sur l'alternance (pourcentage), le nombre d'essais nécessaires à l'atteinte du critère en acquisition et en rétention, le nombre de réponses correctes sur les 5 et sur les 10 premiers essais de la rétention sont comparés entre les groupes (effet TRAITEMENT) par des analyses de variance (ANOVA) suivies de comparaisons des groupes pris deux à deux (test F de Fisher). Les performances de l'acquisition entre les groupes ont été comparées en prenant le nombre de réponses correctes enregistrées sur les 20 premiers essais ou l'équivalent pour les animaux ayant atteint le critère en moins de 20 essais.

### Résultats :

① Acquisition de la discrimination :
Nombre de réponses correctes sur les 20 premiers essais de l'épreuve

| | |
|---|---|
| Tianeptine | 12,75 ± 0,77 |
| Isomère (+) | 14,83 ± 0,75 |
| Placebo | 13,58 ± 0,86 |

Les résultats obtenus avec l'isomère (+) sont supérieurs à ceux obtenus avec le groupe témoin et à ceux obtenus avec la tianeptine racémique.
② Rétention de la discrimination :

*Essais au critère* :
Il existe une différence significative entre les groupes. Les groupes expérimentaux ont des performances supérieures à celles du groupe placebo. L'amélioration la plus importante est observée sur l'isomère (+) :

| | |
|---|---|
| Tianeptine | 8,58 ± 0,60 essais |
| Isomère (+) | 7,83 ± 0,74 essais |
| Placebo | 11,58 ± 1,07 essais |

Les résultats obtenus avec l'isomère (+) sont statistiquement supérieurs (p < 0,01) à ceux obtenus avec le groupe témoin.
*Réponses correctes sur 5 et 10 essais* :
L'effet traitement est significatif dès les cinq premiers essais et au cours des dix premiers essais de la rétention.
Sur les cinq premiers essais, seul **l'isomère (+) a des performances significativement supérieures (p<0,01) à celles du groupe placebo (3,92 ± 0,15 vs 3,0 ± 0,21). La tianeptine n'a pas d'effet**.
Sur les dix premières réponses, les groupes expérimentaux ont des performances supérieures (p<0,05) à celles du groupe placebo. **L'isomère (+) a des performances supérieures (p<0,02) à celles du groupe tianeptine**.

### Conclusion :

**Au cours d'un test pharmacologique reconnu comme prédictif de l'activité promnésique d'un composé, l'isomère (+) a montré une efficacité :**
- **au niveau de l'acquisition, l'isomère (+) a tendance à augmenter le nombre de réponses correctes ;**
- **au niveau de la rétention : essais au critère, réponses correctes sur les5 premiers ou les 10 premiers essais, I'isomère (+) a un effet positif et un effet supérieur à celui de la tianeptine racémique.**

**L'isomère (+) est capable d'augmenter la mémoire des animaux et est donc susceptible d'améliorer les troubles mnésiques chez l'homme.**

### Exemple 3 : Composition pharmaceutique

Comprimés dosés à 12,5 mg d'isomère (+) de tianeptine Formule de préparation pour 1000 comprimés

| | |
|---|---|
| Isomère (+) de la tianeptine | 12,5 g |
| Amidon de blé | 15 g |
| Amidon de maïs | 15 g |
| Lactose | 65 g |
| Stéarate de magnésium | 2 g |
| Silice | 1 g |
| Hydroxypropylcellulose | 2 g |

## Revendications

1. Utilisation de l'isomère (+) du composé de formule (I), ou d'un de ses sels d'addition à un acide ou à une base pharmaceutiquement acceptable, pour l'obtention de médicaments destinés au traitement des troubles mnémo-cognitifs :

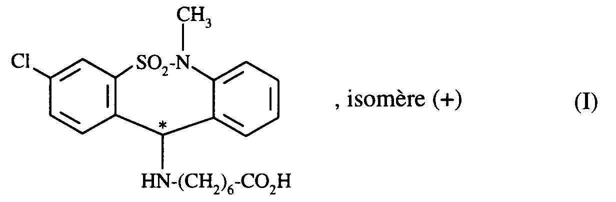

2. Utilisation de l'isomère (+) du composé de formule (I) selon la revendication 1 sous forme de sel de sodium, pour l 'obtention de médicaments destinés au traitement des troubles mnémo-cognitifs.

3. Composition pharmaceutique pour le traitement des troubles mnémo-cognitifs contenant comme principe actif l'isomère (+) du composé de formule (I) selon la revendication 1 ou un de ses sels d'addition à un acide ou à une base pharmaceutiquement acceptable.
